# EUROPEAN PATENT APPLICATION

(11) **EP 2 518 049 A2**
(43) Date of publication of application: **31.10.2012**
(21) Application number: 12174501.2
(22) Date of filing: 12.12.2007
(51) Int. Cl.: C07C 219/22, C07D 309/08, A61K 31/222, A61K 31/351, A61P 29/00

(54) **Aminoalcohol derivatives and their therapeutic use**

(30) Priority: 12.12.2006 GB 0624757
(62) Divisional of application: 07848496.1
(71) Applicant: Biocopea Limited, London Greater London EC4A 1BN (GB)
(72) Inventor: Walmsley, Andrea, Saffron Walden, Essex CM10 1XL (GB); Lasterra, Elena, Cambridge, Cambridgeshire CB4 OEY (GB)
(74) Representative: Stevens, Fiona

(57) **Abstract**

A compound of formula (1) 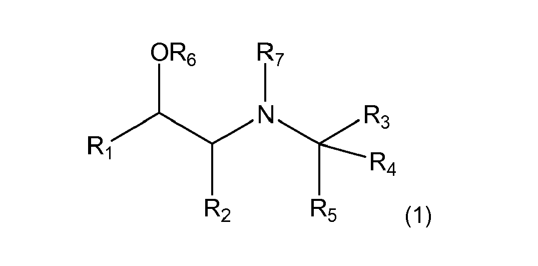 including pharmaceutically acceptable salts thereof, wherein:
R₁ is aryl or heteroaryl optionally substituted with R₈;
R₂ is H or alkyl or CH₂ (when forming part of a ring with R₃, R₄ or R₅);
R₃ is H, alkyl, CH₂OH or CH₂OR₆ and can be part of a ring with R₂;
R₄ is H, alkyl, CH₂OH or CH₂OR₆ and can be part of a ring with R₂;
R₅ is H, alkyl, CH₂OH or CH₂OR₆ and can be part of a ring with R₂;
R₆ is alkyl, COH, COOR₉, CON(R₉)₂, COR₉, COR₁₀, COR₁₁, P(O)ₙR₉, P(O)ₙR₁₀ S(O)ₙR₁₀ or S(O)ₙR₉ and can be part of a ring with R₂, R₃, R₄ or R₅;
R₇ is H, alkyl, COOR₉, COOR₁₁, COR₉ or CON(R₉)_{2,} and can be part of a ring with R₂, R₃, R₄, R₅ or R₆;
R₈ is alkyl, CF₃, OR₉, OCOR₉, CONH₂, CN, F, Cl, Br, I, N(R₉)₂, NO₂, NHCHO, NHCONH₂, NHSO₂R₉, CON(R₉)₂, S(O)ₙR₉, CH₂OH or OCON(R₉)₂;
R₉ is H, alkyl or cycloalkyl;
R₁₀ is aryl or heteroaryl optionally substituted with R₈ or a four to seven membered ring optionally substituted with R₈ and containing one or more additional heteroatoms selected from O, S(O)ₙ and NR₉;
R₁₁ is alkyl optionally substituted with R₈ or R₁₀; and
n is 0, 1 or 2;
provided that when R₃, R₄ or R₅ is CH₂OH then R₆ is not H, and that when R₇ is H and R₃, R₄ and R₅ are alkyl then R₆ is not H.

## Description

### Field of the Invention

This invention relates to novel aminoalcohol derivatives which are inhibitors of cytokines and possess anti-inflammatory properties as well as work at reducing pain in pain conditions where cytokines are involved. The present invention also relates to stereoisomers and pharmaceutical formulations of these compounds.

### Background of the Invention

Immune-driven inflammatory events are a significant cause of many chronic inflammatory diseases where prolonged inflammation causes tissue destruction and results in extensive damage and eventual failure of the effected organ. The cause of these diseases is unknown, so are often called autoimmune, as they appear to originate from an individual's immune system turning on itself. Conditions include those involving multiple organs, such as systemic lupus erythematosus (SLE) and scleroderma. Other types of autoimmune disease can involve specific tissues or organs such as the musculoskeletal tissue (rheumatoid arthritis, ankylosing spondylitis), gastro-intestinal tract, (Crohn's disease and ulcerative colitis), the central nervous system (Alzheimer's, multiple sclerosis, motor neurone disease, Parkinson's disease and chronic fatigue syndrome), pancreatic beta cells (insulin dependent diabetes mellitus), the adrenal gland (Addison's disease), the kidney (Goodpasture's syndrome, IgA nephropathy, interstitial nephritis), exocrine glands (Sjogren's syndrome and autoimmune pancreatitis) and skin (psoriasis and atopic dermatitis).

In addition, there are chronic inflammatory diseases whose aetiology is more or less known but whose inflammation is also chronic and unremitting. These also exhibit massive tissue/organ destruction and include conditions such as osteoarthritis. These conditions are a major cause of illness in the developing world and poorly treated by current therapies.

Inflammation of skin structures (dermatitis) is a common set of conditions These diseases are treated using a wide array of therapies, many of which have very severe side-effects.

Current disease-modifying treatments (if any), for immune-driven conditions, include neutralising antibodies, cytotoxics, corticosteriods, immunosuppressants, antihistamines and antimuscarinics. These treatments are often associated with inconvenient routes of administration and severe side-effects leading to compliance issues. Moreover, certain drug classes are only effective for certain types of inflammatory diseases; e.g. antihistamines for rhinitis.

W02006/027579 discloses compounds of formula (1), below, wherein, *inter alia,* R₁ is optionally substituted aryl or heteroaryl, CR₃R₄R₅ is alkyl or part of a ring with R₂, and R₆ and R₇ are hydrogen.

### Summary of the Invention

According to a first aspect of this invention, novel compounds are of general formula (1) 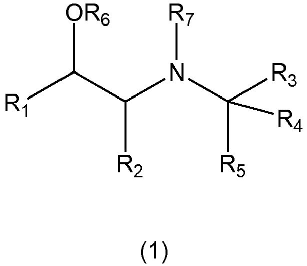 wherein:
R₁ is aryl or heteroaryl optionally substituted with R₈;
R₂ is H or alkyl or CH₂ (when forming part of a ring with R₃, R₄ or R₅);
R₃ is H, alkyl, CH₂OH or CH₂OR₆ and can be part of a ring with R₂;
R₄ is H, alkyl, CH₂OH or CH₂OR₆ and can be part of a ring with R₂;
R₅ is H, alkyl, CH₂OH or CH₂OR₆ and can be part of a ring with R₂;
R₆ is H, alkyl, COH, COOR₉, CON(R₉)₂, COR₉, COR₁₀, COR₁₁, P(O)ₙR₉, P(O)ₙR₁₀, S(O)ₙR₁₀ or S(O)ₙR₉ and can be part of a ring with R₂, R₃, R₄ or R₅;
R₇ is H, alkyl, COOR₉, COOR₁₁, COR₉ or CON(R₉)₂ and can be part of a ring with R₂, R₃, R₄, R₅ or R₆;
R₈ is alkyl, CF₃, OR₉, OCOR₉, CONH₂, CN, F, Cl, Br, I, N(R₉)₂, NO₂, NHCHO, NHCONH₂, NHSO₂R₉, CON(R₉)₂, S(O)ₙR₉, CH₂OH or OCON(R₉)₂;
R₉ is H, alkyl or cycloalkyl;
R₁₀ is aryl or heteroaryl (optionally substituted with R₈) or a four to seven membered ring (which can be optionally substituted with R₈ and can contain one or more additional heteroatoms selected from O, S(O)ₙ and NR₉);
R₁₁ is alkyl optionally substituted with R₈ or R₁₀; and
n is 0, 1 or 2;
provided that when R₃, R₄ or R₅ are CH₂OH then R₆ is not H, and that when R₇
is H and R₃, R₄ and R₅ are alkyl, then R₆ is not H.

It will be understood that reference to compounds of the invention refers to salts, e.g. the hydrochloride, metabolites and pro-drugs thereof, as well as any diastereomers and enantiomers of (I), and also solvates, hydrates and polymorphs.

According to a second aspect of the invention, the compounds are used for the treatment of a condition associated with T-cell proliferation or that is mediated by pro-and/or anti-inflammatory cytokines.

### Description of Preferred Embodiments

As used herein, "alkyl", "aryl", "cycloalkyl" and "heteroaryl" have meanings that will be understood by those in the art. They may include up to 6 or 12 C atoms and 4 to 7 ring atoms. Any ring may be saturated or unsaturated. For example, R₁ may be phenyl or halophenyl. R₆ may form an ester.

A diastereomer or enantiomer of (1) may have little or no activity at the α or β adrenoceptors. This activity may be determined by use of the appropriate in *vitro* assay.

Compounds of formula (1) according to the invention can be used to treat inflammatory diseases including, but not exclusive to, autoimmune diseases involving multiple organs, such as systemic lupus erythematosus (SLE) and scleroderma, specific tissues or organs such as the musculoskeletal tissue (rheumatoid arthritis, ankylosing spondylitis), gastro-intestinal tract, (Crohn's disease and ulcerative colitis), the central nervous system (Alzheimer's, multiple sclerosis, motor neurone disease, Parkinson's disease and chronic fatigue syndrome), pancreatic beta cells (insulin dependent diabetes mellitus), the adrenal gland (Addison's disease), the kidney (Goodpasture's syndrome, IgA nephropathy, interstitial nephritis) exocrine glands (Sjogren's syndrome and autoimmune pancreatitis) and skin (psoriasis and atopic dermatitis), chronic inflammatory diseases such as osteoarthritis, periodontal disease, diabetic nephropathy, diabetic ulceration, retinopathy, chronic obstructive pulmonary disease, artherosclerosis, graft versus host disease, chronic pelvic inflammatory disease, endometriosis, chronic hepatitis and tuberculosis, IgE mediated (Type I) hypersensitivities such as rhinitis, asthma, anaphylaxis,dermatitis and ophthalmic diseases. Dermatitis conditions include; actinic keratosis, acne rosacea, acne vulgaris, allergic contact dermatitis, angioedema, atopic dermatitis, bullous pemiphigoid, cutaneous drug reactions, erythema multiforme, lupus erythematosus, photodermatitis, psoriasis, psoriatic arthritis, scleroderma and urticaria. Ophthalmic diseases include age-related macular degeneration (ARMD), dry eye, uveitis and glaucoma.

These compounds may be used according to the invention when the patient is also administered or in combination with another therapeutic agent selected from corticosteroids (examples including cortisol, cortisone, hydrocortisone, dihydrocortisone, fludrocortisone, prednisone, prednisolone, deflazacort, flunisolide, beconase, methylprednisolone, triamcinolone, betamethasone, and dexamethasone), disease modifying anti-rheumatic drugs (DMARDs) (examples including, azulfidine, aurothiomalate, bucillamine, chlorambucil, cyclophosphamide, leflunomide, methotrexate, mizoribine, penicillamine and sulphasalazine), immunosuppressants (examples including azathioprine, cyclosporine and mycophenolate) COX inhibitors (examples including aceclofenac, acemetacin, alcofenac, alminoprofen, aloxipirin, amfenac, aminophenazone, antraphenine, aspirin, azapropazone, benorilate, benoxaprofen, benzydamine, butibufen, celecoxib, chlorthenoxacine, choline salicylate, chlometacin, dexketoprofen, diclofenac, diflunisal, emorfazone, epirizole, etodolac, feclobuzone, felbinac, fenbufen, fenclofenac, flurbiprofen, glafenine, hydroxylethyl salicylate, ibuprofen, indometacin, indoprofen, ketoprofen, ketorolac, lactyl phenetidin, loxoprofen, mefenamic acid, metamizole, mofebutazone, mofezolac, nabumetone, naproxen, nifenazone, oxametacin, phenacetin, pipebuzone, pranoprofen, propyphenazone, proquazone, rofecoxib, salicylamide, salsalate, sulindac, suprofen, tiaramide, tinoridine, tolfenamic acid and zomepirac), neutralising antibodies (examples including etanercept and infliximab) and antibiotics (examples including, doxycycline and minocycline).

Compounds of formula (I) exhibit analgesic activity in animal models. The activity of these compounds may be determined by the use of the appropriate *in vivo* assay.

This invention also relates to a method of treatment for patients (including man and/or mammalian animals raised in the dairy, meat or fur industries or as pets) suffering from chronic, acute or neuropathic pain; and more specifically, a method of treatment involving the administration of a compound of formula (I) as the active constituent.

Accordingly, the compounds of formula (I) can be used among other things in the treatment of pain conditions such as acute and chronic pain (as well as, but not limited to, pain associated with cancer, surgery, arthritis, dental surgery, trauma, musculo-skeletal injury or disease, visceral diseases, painful bladder syndrome) and migraine headache. Additionally the painful conditions can be neuropathic (postherpetic neuralgia, diabetic neuropathy, drug-induced neuropathy, HIV-mediated neuropathy, sympathetic reflex dystrophy or causalgia, fibromyalgia, myofacial pain, entrapment neuropathy, phantom limb pain, trigeminal neuralgia). Neuropathic conditions include central pain related to stroke, multiple sclerosis, spinal cord injury, arachnoiditis, neoplasms, syringomyelia, Parkinson's and epilepsia.

It will often be advantageous to use compounds of formula (I) in combination with another drug used for pain therapy. Such another drug may be an opiate or a non-opiate such as baclofen. Especially for the treatment of neuropathic pain, coadministration with gabapentin is preferred. Other compounds that may be used include acetaminophen, a non-steroidal anti-inflammatory drug, a narcotic analgesic, a local anaesthetic, an NMDA antagonist, a neuroleptic agent, an anti-convulsant, an anti-spasmodic, an anti-depressant or a muscle relaxant.

In use, any suitable route of administration can be used. For example, any of oral, topical, parenteral, ocular, rectal, vaginal, inhalation, buccal, sublingual and intranasal delivery routes may be suitable. For this purpose a suitable pharmaceutical composition may be used.

A pharmaceutical composition containing the active ingredient may be in a form suitable for oral use, for example, as tablets, troches, lozenges, aqueous or oily suspensions, dispersible powders or granules, emulsions, hard or soft capsules, or syrups or elixirs. The composition may be in immediate or controlled release form.

Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastointestinal tract and thereby provide a sustained action over a longer period. For example, a time delay material such as glyceryl monostearate or glyeryl distearate may be employed. They may also be coated by the techniques described in US4256108, US4166452 or US4265874, to form osmotic therapeutic tablets for control release.

Formulations for oral use may also be presented as hard gelatin capsules where in the active ingredient is mixed with an inert solid diluent, for example calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate polyvinyl-pyrrolidone, gum tragacanth and gum acacia; dispersing or wetting agents may be a naturally occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such a polyoxyethylene with partial esters derived from fatty acids and hexitol anhydrides, for example polyoxyethylene sorbitan monooleate. The aqueous suspensions may also contain one or more preservatives, for example ethyl or n-propyl p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified, for example sweetening, flavouring and colouring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occuring gums, for example gum acacia or gum tragacanth, naturally-occuring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavouring agents.

Syrups and elixirs may be formulated with sweetening agents, for example gycerol, propylene glycol, sorbitol or sucrose. Such formulations may also contain a demulcent, a preservative and flavouring and colouring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be in a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of formula (1) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc containing the compounds of Formula (1) are employed. For purposes of this application, topical application includes mouth washes and gargles.

The amount of active ingredient that may be combined with the carrier materials to produce a single dosage form will vary depending upon the host treated and the particular mode of administration. For example, a formulation intended for the oral administration of humans may vary from about 5 to about 95 percent of the total composition. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy.

Compounds of the general formula (1) may be prepared by any suitable method known in the art and/or by the processes described below. It will be appreciated that where a particular stereoisomer of formula (1) is required, the synthetic processes described herein may be used with the appropriate homochiral starting material and/or isomers maybe resolved from mixtures using conventional separation techniques (e.g. HPLC).

Compounds of formula (1) may be prepared by any suitable method known in the art. In particular, they may be prepared by the following process. It will be appreciated that functional groups, such as amino, hydroxyl or carboxyl groups, present in the various compounds described, and which it is desired to retain, may need to be in protected form before any reaction is initiated. In such instances, removal of the protecting group may be the final step in a particular reaction. Suitable protecting groups for such functionality will be apparent to those skilled in the art. For specific details see "Protective Groups in Organic Synthesis", Wiley Interscience, TW Greene, PGM Wuts.

A process for preparing compounds of general formula (1) comprises conversion of the alcohol moiety in, for example, alcohol (2) or (3), by reaction with an activated carbonyl derivative (such as an acid chloride). The alcohols and the carbonyl derivatives are either commercially available or readily obtained from commercially available materials by people who are skilled in the art of synthetic organic chemistry.

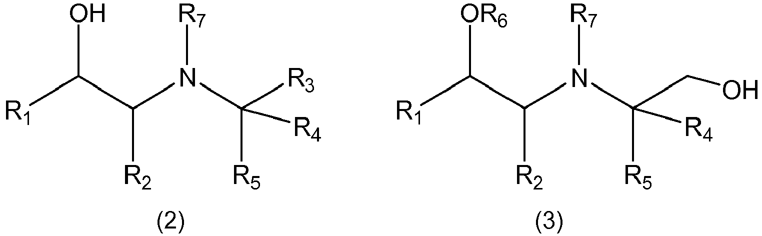

Any mixtures of final products or intermediates obtained can be separated on the basis of the physico-chemical differences of the constituents, in known manner, into the pure final products or intermediates, for example by chromatography, distillation, fractional crystallization, or by formation of a salt if appropriate or possible under the circumstances.

The following numbered embodiments represent preferred embodiments of the present invention:

### Numbered Embodiments

1. A compound of formula (1) 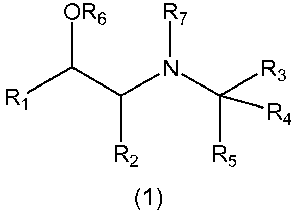 Including pharmaceutically acceptable salts thereof, wherein:
   R₁ is aryl or heteroaryl optionally substituted with R₈;
   R₂ is H or alkyl or CH₂ (when forming part of a ring with R₃, R₄ or R₅);
   R₃ is H, alkyl, CH₂OH or CH₂OR₆ and can be part of a ring with R₂;
   R₄ is H, alkyl, CH₂OH or CH₂OR₆ and can be part of a ring with R₂;
   R₅ is H, alkyl, CH₂OH or CH₂OR₆ and can be part of a ring with R₂;
   R₆ is H, alkyl, COH, COOR₉, CON(R₉)₂, COR₉, COR₁₀, COR₁₁, P(O)ₙR₉, P(O)ₙR₁₀ S(O)ₙR₁₀ or S(O)ₙR₉ and can be part of a ring with R₂, R₃, R₄ or R₅;
   R₇ is H, alkyl, COOR₉, COOR₁₁, COR₉ or CON(R₉)₂, and can be part of a ring with R₂, R₃, R₄, R₅ or R₆;
   R₈ is alkyl, CF₃, OR₉, OCOR₉, CONH₂, CN, F, Cl, Br, I, N(R₉)₂, NO₂, NHCHO, NHCONH₂, NHSO₂R₉, CON(R₉)₂, S(O)ₙR₉, CH₂OH or OCON(R₉)₂;
   R₉ is H, alkyl or cycloalkyl;
   R₁₀ is aryl or heteroaryl (optionally substituted with R₈) or a four to seven membered ring (which is optionally substituted with R₈ and can contain one or more additional heteroatoms selected from the list O, S(O)ₙ and NR₉);
   R₁₁ is alkyl optionally substituted with R₈ or R₁₀; and
   n is 0, 1 or 2;
   provided that when R₃, R₄ or R₅ is CH₂OH then R₆ is not H, and that when R₇ is H and R₃, R₄ and R₅ are alkyl then R₆ is not H.
2. A compound of embodiment 1, in the form of a single enantiomer or diastereoisomer.
3. A compound of embodiment 1, which is
   (+)-(*erythro*)-acetic acid 2-tert-butylamino-1-(3-chlorophenyl)-propyl ester,
   (+)-(erythro)-isobutyric acid 2-tert-butylamino-1-(3-chlorophenyl)-propyl ester,
   (+)-(*erythro*)-3-methoxy-propionic acid 2-tert-butylamino-1-(3-chlorophenyl)-propyl ester or
   (+)-(erythro)-tetrahydropyran-4-carboxylic acid 2-tert-butylamino-1-(3-chlorophenyl)-propyl ester.
4. A compound of any of embodiments 1 to 3, for the treatment or prevention of a condition associated with T-cell proliferation or that is mediated by pro- and/or anti-inflammatory cytokines.
5. A compound of embodiment 4, wherein the condition is a chronic degenerative disease such as rheumatoid arthritis, osteoarthritis or osteoporosis.
6. A compound of embodiment 4, wherein the condition is a chronic demyelinating disease such as multiple sclerosis.
7. A compound of embodiment 4, wherein the condition is a respiratory disease such as asthma or chronic obstructive pulmonary disease.
8. A compound of embodiment 4, wherein the condition is an inflammatory bowel disease (IBD) such as ulcerative colitis or Crohn's disease.
9. A compound of embodiment 4, wherein the condition is a dermatological condition such as psoriasis, scleroderma or atopic dermatitis.
10. A compound of embodiment 4, wherein the condition is a dental disease such as periodontal disease or gingivitis.
11. A compound of embodiment 4, wherein the condition is diabetic nephropathy, lupus nephritis, IgA nephropathy or glomerulonephritis.
12. A compound of embodiment 4, wherein the condition is systemic lupus erythematosus (SLE).
13. A compound of embodiment 4, wherein the condition is graft vs host disease.
14. A compound of embodiment 4, wherein the condition is an ophthalmic disease such as ARMD, dry eye, uveitis and glaucoma.
15. A compound of embodiment 4, wherein the condition is a pain condition.
16. A compound of embodiment 15, wherein the pain condition is chronic pain such as chronic back pain, malignant pain, chronic headache (including migraine and cluster headaches) or arthritic pain.
17. A compound of embodiment 15, wherein the pain condition is acute pain such as post-operative pain, post-traumatic pain or acute disease-induced pain.
18. A compound of embodiment 15, wherein the pain condition is neuropathic pain.
19. A pharmaceutical composition for use in therapy, comprising a compound of any of embodiments 1 or 3 and a pharmaceutically acceptable diluent or carrier.
20. Use of a compound of any of embodiments 1 to 3, for the manufacture of a medicament for the treatment or prevention of a condition as defined in any of claims 4 to 18.
21. Use according to embodiment 20, wherein the patient is also administered another therapeutic agent selected from corticosteroids, cytotoxics, antibiotics, immunosupressants, non-steroidal anti-inflammatory drug, a narcotic analgesic, a local anaesthetic, an NMDA antagonist, a neuroleptic, an anti-convulsant, an anti-spasmodic, an anti-depressant and a muscle relaxant.
22. Use according to embodiment 21, wherein said compound and said another agent are provided in combination.

The following Examples illustrate the invention.

### Example 1

### (+)-(erythro)-Acetic acid 2-tert-butylamino-1-(3-chloro-phenyl)-propyl ester

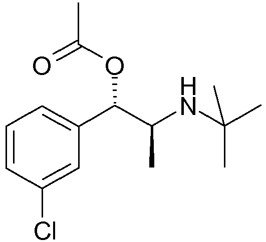

To a solution of (+)-(*erythro)*-2-tert-butylamino-1-(3-chloro-phenyl)-propanol (2.0 g, 8.27 mmol) in dichloromethane (50 mL) at room temperature and under N₂ was added N,N-dimethylaminopyridine (1.01 g, 8.27 mmol), triethylamine (5.8 mL, 41.35 mmol), and acetyl chloride (0.57 mL, 9.92 mmol). The resulting orange solution was stirred for 16 h. The reaction was monitored by TLC DCM:MeOH:NEt₃ (98:2:0.1), followed by quenching with aq. Na₂CO₃ (50 mL). The mixture was extracted into dichloromethane (3 x 50 mL) and the combined organic phases were dried (MgSO₄), filtered, and concentrated under reduced pressure to leave an orange oil (1.82 g, 77%). ¹H NMR (400 MHz, CDCl₃) 0.98 (9H, s), 0.99 (3H, bs), 2.12 (3H, s), 3.01-3.02 (1 H, m), 5.51 - 5.53 (1 H, m), 7.23 - 7.31 (4H, m). ¹³C NMR (100 MHz, CDCl₃) 19.6, 21.3, 30.0, 50.9, 51.4, 77.4, 125.4, 127.3, 127.8, 129.3, 134.1, 140.0, 170.4.

### Example 2

### (+)-(erythro)-Isobutyric acid 2-tert-butylamino-1-(3-chloro-phenyl)-propyl ester

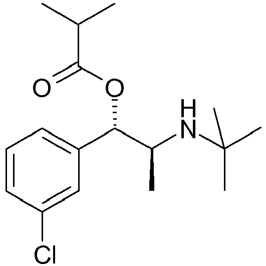

To a solution of (+)-(*erythro)*-2-tert-butylamino-1-(3-chloro-phenyl)-propanol (2.0 g, 8.27 mmol) in dichloromethane (50 mL) at room temperature and under N₂ was added N,N-dimethylaminopyridine (1.01 g, 8.27 mmol), triethylamine (5.8 mL, 41.35 mmol), and isobutyryl chloride (1.04 mL, 9.92 mmol). The resulting orange solution was stirred for 16 h. The reaction was monitored by TLC DCM:MeOH:NEt3 (98:2:0.1), followed by quenching with aq. Na₂CO₃ (50 mL). The mixture was extracted into dichloromethane (3 x 50 mL) and the combined organic phases were dried (MgSO₄), filtered, and concentrated under reduced pressure to leave an orange oil (2.15 g, 83 %). ¹H NMR (400 MHz, CDCl₃) 0.97 (9H, s), 1.05 (3H, d), 1.21 (6H, t), 2.62-2.64 (1 H, m), 3.02 - 3.03 (1 H, m), 5.51 (1 H, d), 7.22 - 7.30 (4H, m). ¹³C NMR (100 MHz, CDCl₃) 19.1, 19.6, 30.0, 34.3, 50.9, 51.6, 79.2, 125.2, 127.1, 127.7, 129.3, 134.1, 141.6, 176.5.

### Example 3

### (+)-(erythro)-3-Methoxy-propionic acid 2-tert-butylamino-1-(3-chloro-phenyl)-propyl ester

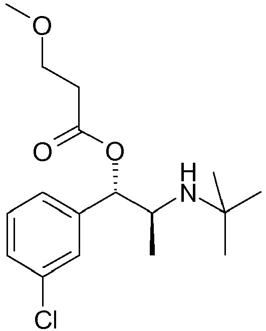

3-Methoxypropionic acid (2.0 g, 19.21 mmol) was dissolved in anhydrous dichloromethane (30 mL) and oxalyl chloride (3.35 mL, 38.42 mmol) was cautiously added. The mixture was stirred at room temperature for 16 h. The solvent was removed under reduced pressure to leave 3-methoxypropionyl chloride as a brown oil. This material was used without any further purification (2.2 g, 94%). ¹H NMR(400 MHz, CDCl₃) 3.08 (2H, t), 3.33 (3H, s), 3.65 (2H, t).

To a solution of (+)-*(erythro)*-2-tert-butylamino-1-(3-chloro-phenyl)-propanol (2.0 g, 8.27 mmol) in dichloromethane (50 mL) at room temperature and under N₂ was added N,N-dimethylaminopyridine (1.01 g, 8.27 mmol), triethylamine (5.8 ml), 41.35 mmol), and 3-methoxypropionyl chloride (1.22 g, 9.92 mmol). The resulting orange solution was stirred for 16 h. The reaction was monitored by TLC DCM:MeOH:NEt₃ (98:2:0.1), followed by quenching with aq. Na₂CO₃ (50 mL). The mixture was extracted into dichloromethane (3 x 50 mL) and the combined organic phases were dried (MgSO₄), filtered, and concentrated under reduced pressure to leave an orange oil (1.2g, 44%). ¹H NMR (400 MHz, CDCl₃) 1.02 (9H, s), 1.04 (3H, d), 2.67 (2H, t), 3.00 -3.03 (1 H, m), 3.35 (3H, s), 3.68 - 3.70 (2H, m), 5.58 (1 H, d), 7.22 - 7.32 (4H, m). ¹³C NMR (100 MHz, CDCl₃) 19.4, 30.0, 35.5, 51.0, 51.5, 58.8, 68.1, 79.7, 125.2, 127.1, 127.7, 129.3, 134.1, 141.9, 171.2.

### Example 4

### (+)-(erythro)-Tetrahydropyran-4-carboxylic acid 2-tert-butylamino-1-(3-chlorophenyl)-propyl ester

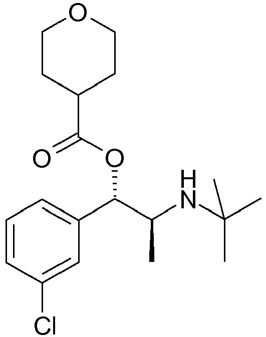

Tetrahydropyran-4-carnboxylic acid (0.92g, 7.07 mmol) was dissolved in thionyl chloride (5.7 mL, 77.8 mmol) and refluxed for 2.5 h. After this time the mixture was cooled to room temperature and evaporated to dryness. Toluene (50 mL) was added and the resulting oil and the mixture was evaporated to dryness to give tetrahydropyran-4-carbonyl chloride as a brown oil. This material was used without any further purification (1 g, 95%). ¹H NMR (400 MHz, CDCl₃) 1.85 - 1.90 (2H, m), 1.91 - 2.0 (2H, m), 2.94 - 2.96 (1 H, m), 3.42 - 3.43 (2H, m) 3.97 - 4.00 (2H, s).

To a solution of (+)-*(erythro)*-2-tert-butylamino-1-(3-chloro-phenyl)-propanol (1.2 g, 4.96 mmol) in dichloromethane (50 mL) at room temperature and under N₂ was added N,N-dimethylaminopyridine (0.6 g, 4.96 mmol), triethylamine (3.45 mL, 24.8 mmol), and tetrahydropyran-4-carbonyl chloride (0.89 g, 5.96 mmol). The resulting orange solution was stirred overnight. The reaction was monitored by TLC DCM:MeOH:NEt₃ (98:2:0.1), followed by quenching with aq. Na₂CO₃ (50 mL). The mixture was extracted into dichloromethane (3 x 50 mL) and the combined organic phases were dried (MgSO₄), filtered, and concentrated under reduced pressure to leave orange oil (0.72g). ¹H NMR (400 MHz, CDCl₃) 0.97 (9 H, s) 1.04 (3 H, d) 1.80-1.87 (4 H, m) 2.62 - 2.64 (1 H, m) 3.03 - 3.04 (1 H, m) 3.42 - 3.45 (2 H, m) 3.95 - 3.96 (2 H, m) 5.54 (1 H d) 7.22 - 7.28 (4 H, m). ¹³C (100 MHz, CDCl₃) 19.5,28.4,29.9, 40.4, 51.0, 51.6, 67.2, 79.4, 125.2, 127.1, 127.8, 129.4, 134.1, 141.3, 173.7.

### Carrageenan paw oedema assay

Fasted (18 hour) male Wistar rats (105-130 g) were weighed and a basal mercury plethysmometer reading was taken of the right hind paw by submerging the paw in the mercury up to the tibiotarsal joint. Subsequently, vehicles, reference items and test articles were administered by oral gavage (10 ml/kg). Half an hour after treatment, 0.1 ml of 2% carrageenan in 0.9% saline was injected into the subplanatar area of the right hind paw. The right paw was measured again with the plethysmometer at 1, 2, 3, 4 and 5 hours after carrageenan administration. Paw volume effects was expressed as the area under the curve for paw volume over time. Activity (inhibition of paw volume) was expressed as the % antiinflammatory activity versus the vehicle control.

The compounds of Examples 1 to 4 showed an anti-inflammatory effect against intraplantar carrageenan-induced paw oedema.

## Claims

1. A compound of formula (1) 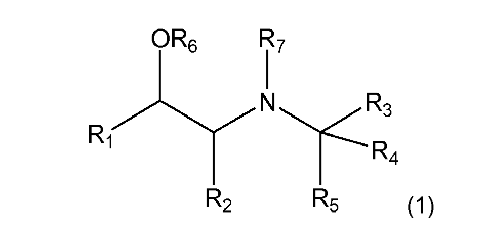 including pharmaceutically acceptable salts thereof, wherein:
R₁ is aryl or heteroaryl optionally substituted with R₈;
R₂ is H or alkyl or CH₂ (when forming part of a ring with R₃, R₄ or R₅);
R₃ is H, alkyl, CH₂OH or CH₂OR₆ and can be part of a ring with R₂;
R₄ is H, alkyl, CH₂OH or CH₂OR₆ and can be part of a ring with R₂;
R₅ is H, alkyl, CH₂OH or CH₂OR₆ and can be part of a ring with R₂;
R₆ is alkyl, COH, COOR₉, CON(R₉)₂, COR₉, COR₁₀, COR₁₁, P(O)ₙR₉, P(O)ₙR₁₀ S(O)ₙR₁₀ or
S(O)ₙR₉ and can be part of a ring with R₂, R₃, R₄ or R₅;
R₇ is H, alkyl, COOR₉, COOR₁₁, COR₉ or CON(R₉)₂, and can be part of a ring with R₂, R₃, R₄, R₅ or R₆;
R₈ is alkyl, CF₃, OR₉, OCOR₉, CONH₂, CN, F, Cl, Br, I, N(R₉)₂, NO₂, NHCHO, NHCONH₂, NHSO₂R₉, CON(R₉)₂, S(O)ₙR₉, CH₂OH or OCON(R₉)₂;
R₉ is H, alkyl or cycloalkyl;
R₁₀ is aryl or heteroaryl optionally substituted with R₈ or a four to seven membered ring optionally substituted with R₈ and containing one or more additional heteroatoms selected from O, S(O)ₙ and NR₉;
R₁₁ is alkyl optionally substituted with R₈ or R₁₀; and
n is 0, 1 or 2;
provided that when R₃, R₄ or R₅ is CH₂OH then R₆ is not H, and that when R₇ is H and R₃,
R₄ and R₅ are alkyl then R₆ is not H.

2. A compound according to claim 1, in the form of a single enantiomer or diastereoisomer.

3. A compound according to claim 1 or 2, wherein
R₁ is aryl optionally substituted with R₈;
R₂ is H or alkyl;
R₃ is H, alkyl, CH₂OH or CH₂OR₆;
R₄ is H, alkyl, CH₂OH or CH₂OR₆;
R₅ is H, alkyl, CH₂OH or CH₂OR₆;
R₆ is alkyl, COH, COOR₉, CON(R₉)₂, COR₉, COR₁₀, COR₁₁, P(O)ₙR₉, P(O)ₙR₁₀S(O)ₙR₁₀ or S(O)ₙR₉;
R₇ is H, alkyl, COOR₉, COOR₁₁, COR₉ or CON(R₉)₂;
R₈ is alkyl, CF₃, OH, OCOH, OR₉, CONH₂, CN, F, Cl, Br, 1, NH₂, NO₂, NHCHO, NHCONH₂, NHSO₂H, CONH₂, S(O)ₙH, CH₂OH or OCONH₂;
R₉ is H, alkyl or cycloalkyl;
R₁₀ is a four to seven membered ring optionally substituted with R₈ and containing one or more additional heteroatoms selected from O, S(O)ₙ and NR₉;
R₁₁ is alkyl optionally substituted with R₈ or R₁₀; and
n is 0, 1 or 2.

4. A compound according to any preceding claim, wherein
R₁ is aryl optionally substituted with R₈;
R₂ is H or alkyl;
R₃ is H, alkyl, or CH₂OH;
R₄ is H, alkyl, or CH₂OH;
R₅ is H, alkyl, or CH₂OH;
R₆ is COR₉, COR₁₀, or COR₁₁;
R₇ is H, alkyl, COOH, or CONH₂;
R₈ is alkyl, CF₃, OH, OCOH, OR₉, CONH₂, CN, F, Cl, Br, or I;
R₉ is H, alkyl;
R₁₀ is a six membered ring optionally substituted with R₈ and containing one or more additional heteroatoms selected from O, S(O)ₙ and NR₉;
R₁₁ is alkyl optionally substituted with R₈ or R₁₀; and
n is 0, 1 or 2.

5. A compound according to any preceding claim, wherein
R₁ is aryl optionally substituted with R₈;
R₂ is alkyl;
R₃ is alkyl;
R₄ is alkyl;
R₅ is alkyl;
R₆ is COR₉, COR₁₀, or COR₁₁;
R₇ is H;
R₈ is alkyl, OH, OCOH, OR₉, F, Cl, Br, or I;
R₈ is H or alkyl;
R₁₀ is a six membered ring optionally substituted with R₈ and containing one or more O; and
R₁₁ is alkyl optionally substituted with R₈ or R₁₀.

6. A compound according to any preceding claim, wherein
R₁ is aryl optionally substituted with R₈;
R₂ is alkyl;
R₃ is alkyl;
R₄ is alkyl;
R₅ is alkyl;
R₆ is COR₉, COR₁₀, or COR₁₁;
R₇ is H;
R₈ is OCH₃, F, Cl, Br, or I;
R₉ is H or alkyl;
R₁₀ is a six membered ring optionally substituted with R₈ and containing one or more O; R₁₁ is alkyl optionally substituted with R₈ or R₁₀.

7. A compound according to any preceding claim, wherein R₁ is aryl optionally substituted with R₈;
R₂ is alkyl;
R₃ is alkyl;
R₄ is alkyl;
R₅ is alkyl;
R₆ is COR₉, COR₁₀, or COR₁₁,
R₇ is H;
R₈ is OCH₃ or Cl;
R₉ is alkyl;
R₁₀ is a six membered ring optionally substituted with R₈ and containing one or more O; R₁₁ is alkyl optionally substituted with R₈ or R₁₀.

8. A compound according to any preceding claim, wherein R₁ is aryl optionally substituted with R₈;
R₂ is alkyl;
R₃ is alkyl;
R₄ is alkyl;
R₅ is alkyl;
R₆ is COR₉;
R₇ is H;
R₈ is Cl; and
R₉ is alkyl; or
wherein
R₁ is aryl optionally substituted with R₈;
R₂ is alkyl;
R₃ is alkyl;
R₄ is alkyl;
R₅ is alkyl;
R₆ is COR₁₀;
R₇ is H;
R₈ is Cl;
R₉ is alkyl; and
R₁₀ is a six membered ring containing one or more additional heteroatoms selected from O; or
wherein
R₁ is aryl optionally substituted with R₈; R₂ is alkyl;
R₃ is alkyl;
R₄ is alkyl;
R₅ is alkyl;
R₆ is COR₁₁;
R₇ is H;
R₈ is OCH₃ or Cl; and
R₁₁ is alkyl optionally substituted with R₈.

9. A compound according to any preceding claim, wherein the compound is

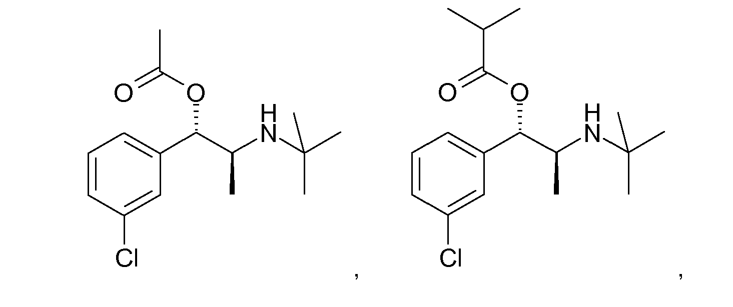

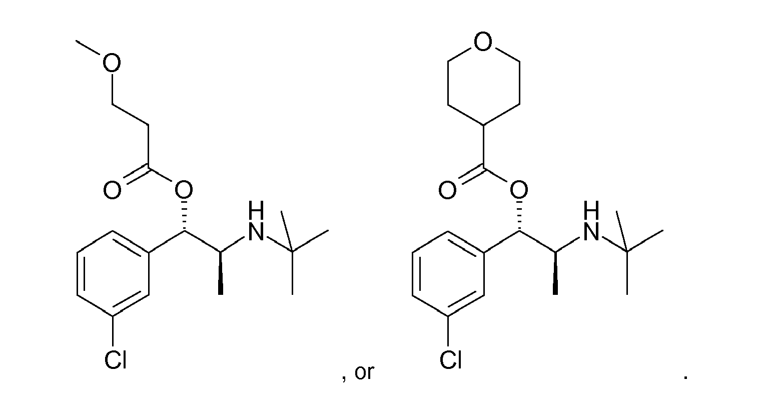

10. A pharmaceutical composition comprising a compound as defined in any preceding claim and a pharmaceutically acceptable diluent or carrier.

11. A compound according to any of claims 1-9 or a pharmaceutical composition according to claim 10 for the treatment or prevention of a condition associated with T-cell proliferation or that is mediated by pro-inflammatory cytokines and/or anti-inflammatory cytokines.

12. A compound for use according to claim 11, wherein the condition is a systemic lupus erythematosus (SLE), a graft vs host disease, a chronic degenerative disease such as rheumatoid arthritis, osteoarthritis or osteoporosis, a chronic demyelinating disease such as multiple sclerosis, a respiratory disease such as asthma or chronic obstructive pulmonary disease, an inflammatory bowel disease (IBD) such as ulcerative colitis or Crohn's disease, a dermatological condition such as psoriasis, scleroderma or atopic dermatitis, a dental disease such as periodontal disease or gingivitis, a diabetic nephropathy, lupus nephritis, IgA nephropathy or glomerulonephritis, an ophthalmic disease such as ARMD, dry eye, uveitis and glaucoma or a pain condition.

13. A compound for use according to claim 12, wherein the pain condition is a chronic pain such as chronic back pain, malignant pain, chronic headache (including migraine and cluster headaches) or arthritic pain; an acute pain such as postoperative pain, post-traumatic pain or acute disease-induced pain; and/or a neuropathic pain.

14. A compound for use according to any of claims 11 to 13, wherein the patient is also administered another therapeutic agent selected from corticosteroids, cytotoxics, antibiotics, immunosupressants, non-steroidal anti-inflammatory drug, a narcotic analgesic, a local anaesthetic, an NMDA antagonist, a neuroleptic, an anti-convulsant, an antispasmodic, an anti-depressant and a muscle relaxant.

15. A compound for use according to claim 14, wherein the compound and the another agent are provided in combination
